(19) Europäisches Patentamt
European Patent Office
Office européen des brevets

(11) Veröffentlichungsnummer: **0 093 332**
**B1**

(12) # EUROPÄISCHE PATENTSCHRIFT

(45) Veröffentlichungstag der Patentschrift: **21.08.85**

(51) Int. Cl.⁴: **C 07 C 120/06**, C 07 C 121/16, C 07 C 120/10

(21) Anmeldenummer: **83103893.0**

(22) Anmeldetag: **21.04.83**

(54) **Verfahren zur Herstellung von Pivalinsäurenitril.**

(30) Priorität: **03.05.82 DE 3216382**

(43) Veröffentlichungstag der Anmeldung: **09.11.83 Patentblatt 83/45**

(45) Bekanntmachung des Hinweises auf die Patenterteilung: **21.08.85 Patentblatt 85/34**

(84) Benannte Vertragsstaaten: **BE CH DE FR GB IT LI NL**

(56) Entgegenhaltungen:
**FR - A - 2 341 562**
**FR - A - 2 418 223**

**CHEMICAL ABSTRACTS, Band 78, 1973, Seite 458, Nr. 29194n, Columbus, Ohio, USA T.I. KOVALENKO et al.: "Catalytic synthesis of aliphatic nitriles from carboxylic acids"**

(73) Patentinhaber: **BAYER AG, Konzernverwaltung RP Patentabteilung, D-5090 Leverkusen 1 Bayerwerk (DE)**

(72) Erfinder: **Hagedorn, Ferdinand, Dr., Domblick 16, D-5090 Leverkusen 3 (DE)**
Erfinder: **Wedemeyer, Karlfried, Dr., Bilharzstrasse 7, D-5000 Köln 80 (DE)**
Erfinder: **Häusser, Friedrich, Dr., Bodelschwinghstrasse 22, D-4150 Krefeld 1 (DE)**
Erfinder: **Schwarz, Hans-Helmut, Dr., Rather Strasse 90, D-4150 Krefeld 1 (DE)**
Erfinder: **Tragler, Dieter, Mauritzstrasse 55, D-4150 Krefeld 11 (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).



BUNDESDRUCKEREI BERLIN

**Beschreibung**

Die Erfindung betrifft ein auch großtechnisch anwendbares Verfahren zur Herstellung von Pivalinsäurenitril durch katalysierte Umsetzung von Pivalinsäure mit Ammoniak in der Gasphase; das neue Verfahren liefert Pivalinsäurenitril mit hoher Ausbeute und Reinheit.

Bekannt ist die Herstellung von Pivalinsäurenitril durch katalysierte Anlagerung von Blausäure an Isobuten in der Gasphase (NL 6 916 495). Dieses Verfahren ist mit dem Nachteil behaftet, daß die Standzeit der benutzten Katalysatoren kurz ist und der ohnehin mit nur 55% beginnende Isobuten-Umsatz bereits nach 3 Stunden auf 49% abgefallen ist. Ein weiterer Nachteil dieses Verfahrens ist die Tatsache, daß als Nebenprodukt Acetonitril entsteht, so daß zur Gewinnung eines reinen Pivalonitrils eine aufwendige Reinigung des Rohnitrils erforderlich ist.

Gemäß US-PS 4 203 917 kann man durch Gasphasen-Reaktion von N-tert.-Butylformamid an Ammonoxidationskatalysatoren, wie z. B. Bismutphosphomolybdat, bei relativ hoher Temperatur (550° C) Pivalinsäurenitril herstellen (s. Spalte 6, Beispiel 3). Die Ausbeute und die Selektivität liegen mit 45,4% bzw. 41,3% allerdings so niedrig, daß dieses Verfahren für die technische Pivalinsäurenitril-Herstellung als ungeeignet anzusehen ist.

Es ist ferner bekannt, durch Wasserabspaltung aus Pivalinsäureamid mit geeigneten Chemikalien wie z. B. Phosphorpentoxid [Liebigs Ann. Chem. 173, 374 (1874)] oder aus Trimethylacetaldoxim durch Erhitzen in Hexamethylphosphorsäuretriamid [Tetrahedron 30, (15), 2509—14 (1974)] Pivalinsäurenitril herzustellen [siehe auch Houben—Weyl, Methoden der Org. Chem., 4. Auflage, Bd. 8, S. 325 und 330 (1952)].

Diese Verfahren sind zum Teil umständlich und teuer oder aus Sicherheitsgründen technisch nicht durchführbar, außerdem müßten die Ausgangsmaterialien z. B. aus Pivalinsäure oder Pivalinaldehyd erst hergestellt werden.

Des weiteren ist die Herstellung von geradkettigen aliphatischen Nitrilen aus geradkettigen Carbonsäuren und Ammoniak z. B. an Aluminiumoxid-Kontakten in der Gasphase mehrfach beschrieben. Valeriansäurenitril kann z. B. mit optimalen Ausbeuten von 97% bei 520° C hergestellt werden [T. I. Kovalenko et al., Zh. Prikl. Khim. 45, (8), 1824—27 (1972), engl. Übersetzung, S. 1901—03]. Bei dieser Temperatur wird jedoch die hoch verzweigte Pivalinsäure bereits weitgehend zu Isobuten, Wasser und CO zersetzt, während Pivalinsäurenitril bei 520° C unter Blausäure-Abspaltung Isobuten liefert.

Dieses vorbekannte Verfahren ist somit zur analogen Herstellung von Pivalinsäurenitril nicht anwendbar. Die Umsetzung von Pivalinsäure mit Ammoniak bei hohen Temperaturen in der Gasphase unter Bildung von Pivalinsäurenitril ist bisher, soweit sich feststellen ließ, nicht in der Literatur beschrieben worden. Es bestand vielmehr für den Fachmann das Vorurteil, daß man Pivalinsäurenitril durch Gasphasenreaktion von Pivalinsäure mit Ammoniak bei höheren Temperaturen nicht mit befriedigender Ausbeute erhalten kann.

Es wurde nun gefunden, daß man Pivalinsäurenitril mit überraschend hoher Reinheit und mit hoher Ausbeute bei fast vollständigem Säure-Umsatz erhält, wenn man Pivalinsäure mit Ammoniak, gegebenenfalls in Anwesenheit von Wasserdampf, an einem Aluminiumoxid-Kontakt in der Gasphase im Temperaturbereich von 370—430° C umsetzt.

Die erfindungsgemäße Umsetzung kann durch das folgende Reaktionsschema wiedergegeben werden:

$$(CH_3)_3C-COOH + NH_3 \xrightarrow[370-430^\circ C]{[\gamma\text{-}Al_2O_3]} (CH_3)_3C-CN + 2\,H_2O$$

Pivalinsäure — Pivalonitril

Pivalinsäure ist durch bekannte Verfahren, z. B. ausgehend von Kohlenmonoxid und Isobuten, oder aus Pinakolon durch Oxidation zugänglich und wird bereits großtechnisch hergestellt. Käufliche Pivalinsäure ist für das vorliegende Verfahren geeignet und bedarf keiner Vorreinigung. Sie wird in flüssiger Form eingesetzt, verdampft und mit Ammoniak am Katalysator zur Reaktion gebracht.

Ammoniak kann gasförmig oder als wäßrige Lösung eingesetzt werden. Wasserdampf stört die Gasphasenreaktion nicht, zumal bei der Umsetzung pro Mol Pivalinsäurenitril 2 Mol Wasser entstehen. Bevorzugt wird gasförmiges Ammoniak eingesetzt, um die zwangsläufig anfallende Wassermenge möglichst gering zu halten. Es können jedoch auch z. B. ca. 15 bis 25%ige wäßrige Ammoniak-Lösungen verwendet werden.

Im allgemeinen werden Pivalinsäure und Ammoniak im Molverhältnis 1 : 1 bis 1 : 3 zur Reaktion gebracht, wobei ein schwacher Überschuß an Ammoniak ausreicht. Bevorzugt wählt man ein Molverhältnis von 1 : 1 bis 1 : 2, besonders bevorzugt ein Verhältnis von 1 : 1,05 bis 1 : 1,6. Bei einem Ammoniakunterschuß erhält man zwar ebenfalls noch befriedigende Nitril-Ausbeuten, wobei jedoch der Pivalinsäureumsatz unvollständig bleiben muß. Diese Arbeitsweise ist unwirtschaftlich und macht eine Wiedergewinnung nicht umgesetzter Pivalinsäure notwendig. Auch ein Molverhältnis Ammoniak/Pivalinsäure größer als 3 ist eher von Nachteil im Hinblick auf die Aufarbeitung der Reaktionsprodukte.

Als Katalysatoren eignen sich besonders $\gamma$-Aluminiumoxide mit einem Gehalt $>99\%$ $Al_2O_3$ und mit

einer spezifischen Oberfläche von 50—350 $m^2/g$. Die Katalysatoren sind als solche bekannt und käuflich (z. B. die Handelsprodukte »D 10-10« der Firma BASF AG, »SCS-79«, »250« oder »350« der Firma Rhône-Poulenc oder »FV 69« der Firma Kali-Chemie).

Für das erfindungsgemäße Verfahren eignen sich insbesondere solche Aluminiumoxide, die möglichst frei von Schwermetalloxiden, insbesondere Eisen-, Chrom-, Mangan-, Molybdän-, Nickel- und Kobaltoxiden sind. So liegt der Gehalt an $Fe_2O_3$ und anderen Schwermetalloxiden bei den als bevorzugt zu wählenden Katalysatoren unter 0,1%.

Die Reaktion wird in der Gasphase an dem fest angeordneten Katalysator durchgeführt. Der Katalysator kann in beliebiger Form eingesetzt werden, z. B. in Form von Kugeln, Strang- oder Zylinder-Preßlingen oder als Schlacke. Zweckmäßig wählt man eine solche Form, die für die Befüllung und Entleerung des Reaktors besonders geeignet erscheint, z. B. ein Kugel-Granulat oder zylindrische Strangpreßlinge. Die käuflichen Katalysatoren bedürfen keiner weiteren Vorbehandlung.

Die Reaktionstemperatur beträgt, wie oben angegeben, 370—430° C. Es war überraschend, daß bereits bei 380° C Pivalinsäurenitril-Ausbeuten >95% erhalten werden können, während vergleichsweise für Valeriansäurenitril bei dieser Temperatur gemäß Kovalenko et al., Zh. Prikl. Khim. (s. oben) lediglich eine Ausbeute von 58,6% erzielt worden ist.

Die Katalysatorbelastung kann in weiten Grenzen variiert werden, wobei insbesondere die hohe Raum-Zeit-Ausbeute, die mit den genannten Katalysatoren erzielbar ist, überraschte. So kann man mit einer Belastung von 650 g Pivalinsäure pro Liter Katalysator und Stunde noch Nitril-Ausbeuten von 94% erzielen, während mit einem Durchsatz von 300 g Pivalinsäure pro Liter Kontakt und Stunde Ausbeuten bis 98% der Theorie erreichbar sind. Bei Durchsätzen von Pivalinsäure >700 g/l/h ist die Nitrilausbeute geringer, da die Verweilzeit zur vollständigen Umsetzung der Säure nicht mehr ausreicht.

Die Verweilzeit ist in diesem Zusammenhang definiert als die Zeiteinheit, die ein bestimmtes Gasvolumen benötigt, um bei Reaktionsbedingungen (Temperatur, Druck) ein von Katalysator leer gedachtes Reaktorvolumen zu durchströmen. Sie beträgt im Falle des erfindungsgemäßen Verfahrens im allgemeinen 0,5 bis 20 Sekunden, bevorzugt 1 bis 12 Sekunden.

Der Einsatz von Wasserdampf ist nicht erforderlich, zumal bei der Reaktion Wasser entsteht; er wirkt sich jedoch nicht nachteilig auf die Umsetzung aus, wenn z. B. wäßrige Ammoniak-Lösung eingesetzt wird.

Das Verfahren wird bevorzugt drucklos durchgeführt, es kann jedoch auch bei schwachem Überdruck oder Unterdruck gearbeitet werden.

Das Verfahren wird bevorzugt kontinuierlich durchgeführt. Auch eine diskontinuierliche Arbeitsweise ist möglich. Das Verfahren kann allgemein so durchgeführt werden, daß man Pivalinsäure und Ammoniak getrennt voneinander verdampft, die Dämpfe auf die notwendige Reaktionstemperatur vorerhitzt und, gegebenenfalls nach Vermischen in einer Vorheizzone, über einen in einem beheizbaren Reaktionsrohr fest angeordneten Aluminiumoxid-Katalysator leitet. Die Reaktionsprodukte Pivalinsäurenitril und Wasser werden abgekühlt und in einem Behälter aufgefangen. Das Pivalinsäurenitril besitzt nur geringe Wasserlöslichkeit und läßt sich leicht vom Wasser abtrennen. Die Wasserphase enthält noch etwas überschüssiges Ammoniak, wenig Pivalinsäurenitril und gegebenenfalls etwas nicht umgesetzte Pivalinsäure als Ammoniumsalz. Diese Stoffe können nach bekannten Methoden, z. B. durch Erhitzen oder Extrahieren mit geeigneten Lösungsmitteln, wiedergewonnen werden, um ein unbelastetes Reaktionswasser zu erhalten. Ein umweltbelastendes Abgas entsteht bei diesem Verfahren nicht.

Für die Durchführbarkeit des erfindungsgemäßen Verfahrens im (groß)technischen Maßstab sind insbesondere solche Apparaturen geeignet, die aus Tantal oder bestimmten Nickelbasislegierungen gefertigt sind. Derartige Nickellegierungen enthalten ca. 56—70% Nickel, 14—23% Chrom und 8—17% Molybdän, neben geringen Mengen (jeweils ≤5%) an anderen Elementen, wie Fe, Co, Nb, Ta, Ti, Al und/oder C (vergleiche Beispielteil).

Das erfindungsgemäß hergestellte Pivalinsäurenitril eignet sich nach Abtrennung der Wasserphase wegen seiner hohen Reinheit bevorzugt für die katalytische Hydrierung zu Neopentylamin (vgl. die gleichzeitig angemeldete deutsche Offenlegungsschrift 3 216 384. Neopentylamin dient seinerseits als Zwischenprodukt zur Herstellung von bestimmten herbizid wirksamen Pflanzenschutzmitteln (vergleiche z. B. DE-OS 2 254 200, US-PS 4 056 527, DE-PS 2 257 344, US-PS 3 950 367, DE-OS 2 351 556, US-PS 3 962 327, DE-OS 3 006 226, DE-OS 3 006 263, DE-OS 3 035 392, DE-OS 3 035 393).

Die nachfolgenden Beispiele sollen zur weiteren Erläuterung der Erfindung dienen.

## Beispiele

### Beispiel 1

In ein beheizbares Reaktionsrohr aus Glas füllt man 90 $cm^3$ eines $\gamma$-Aluminiumoxid-Katalysators mit einer spezifischen Oberfläche von 230 $m^2/g$ und einer Schüttdichte von etwa 0,65 kg/Liter. Über diesen Kontakt leitet man bei 380° C ein vorerhitztes Gasgemisch aus Pivalinsäure (468 g/Liter Katalysator/Stunde) und Ammoniak im Molverhältnis 1 : 1,05. Die heißen Reaktionsgase werden nach Passieren der Katalysatorschicht abgekühlt, wobei Pivalinsäurenitril und Wasser kondensieren und in einem Auffangbehälter zwei Phasen bilden. Die flüssige organische Phase besteht zu 99% aus Pivalinsäurenitril und läßt sich leicht abtrennen. Die Ausbeute, bezogen auf eingesetzte Pivalinsäure, beträgt 96% der Theorie.

Beispiel 2

In ein beheizbares Reaktionsrohr füllt man 1000 $cm^3$ eines $\gamma$-Aluminiumoxid-Katalysators mit einer spezifischen Oberfläche von 80—95 $m^2/g$ und einer Schüttdichte von 0,7 kg/Liter. Man leitet, ähnlich wie im Beispiel 1 beschrieben, gasförmige Pivalinsäure (312 g/Liter Katalysator/Stunde) und gleichzeitig gasförmiges Ammoniak im Molverhältnis 1 : 1,05 bei 420—430° C über den Kontakt. Man erhält nach Kondensation der Reaktionsgase und Phasentrennung das Rohnitril mit einer Ausbeute von 98% der Theorie, bezogen auf eingesetzte Pivalinsäure. Das Rohnitril hat einen Gehalt von 99,4%.

Beispiel 3

Bei Wiederholung des Beispiels 1 mit einem Aluminiumoxid-Katalysator, der 0,025% $Fe_2O_3$ enthält, dessen spezifische Oberfläche 275 $m^2/g$ und dessen Schüttdichte 0,75 kg/l beträgt, erhält man bei Reaktionstemperaturen von 420° C eine Pivalinsäurenitril-Ausbeute von 98% der Theorie, bezogen auf eingesetzte Pivalinsäure.

Die folgenden Versuche gemäß Beispielen 4—6 dienten zur Ermittlung geeigneter Materialien für eine technische Apparatur zur Durchführung des erfindungsgemäßen Verfahrens in großem Maßstab.

Beispiel 4

Beispiel 1 wurde mit 20 g eines $\gamma$-Aluminiumoxid-Katalysators (in einer Laborapparatur aus Glas) wiederholt. Über den Katalysator wurde bei 380° C ein vorgewärmtes Gemisch aus Pivalinsäure (480 g/Liter Katalysator/Stunde) und Ammoniak im Molverhältnis 1 : 1,58 geleitet. Die Anlage wurde 7340 Stunden lang betrieben, ohne daß die Wirksamkeit des Katalysators beeinträchtigt wurde.

Jedoch ist nach einer Betriebszeit von ca. 4000—5000 Stunden eine zunehmende Rußablagerung auf dem Katalysator zu beobachten. Der Katalysator kann jederzeit mit Luftsauerstoff bei Temperaturen von etwa 440—490° C regeneriert werden.

Dieser Versuch zeigt die erreichbaren hohen Katalysatorstandzeiten, wenn kein korrodierbares Metall zugegen ist.

Beispiel 5
(Vergleichsversuch)

Beispiel 4 wurde mit 5275 g eines $\gamma$-Aluminiumoxid-Katalysators in einer halbtechnischen Apparatur aus dem Werkstoff 1.4571 (V4A-Stahl) wiederholt. Bei 380—400° C wurde ein vorerhitztes Gemisch aus Pivalinsäure (314,5 g/Liter Katalysator/Stunde) und Ammoniak im Molverhältnis 1 : 1,6 über den Kontakt geleitet. Nach 1290 Betriebsstunden war die Katalysatorcharge durch Metall- und Kohlenstoffablagerungen für die Reaktion zwischen Pivalinsäure und Ammoniak unwirksam. Auf dem Katalysator wurden die Bestandteile des Werkstoffs 1.4571 gefunden, der durch die Einsatzprodukte in hohem Maße korrosiv angegriffen wird. Eine Regenerierung des Kontaktes war nicht möglich.

Beispiel 6

Beispiel 4 wurde mehrfach derart wiederholt, daß verschiedenartige metallische Werkstoffe von den gasförmigen Reaktanden beansprucht wurden. Dazu wurden entsprechende Werkstoffproben vor dem Katalysator in die Glasapparatur eingebracht. Nach ca. 220 Betriebsstunden war bei den Materialien Tantal, »Inconel 625« und »Hastelloy C-4« kein Einfluß auf die katalytische Wirksamkeit des Kontaktes zu beobachten.

Zum Vergleich: Bei Verwendung der austenitischen Werkstoffe 1.4571 (V4A-Stahl) bzw. 1.4539 war bereits nach 120 Betriebsstunden eine deutliche Desaktivierung des Katalysators durch metallische Ablagerungen zu bemerken.

Bei der großtechnischen Durchführung des erfindungsgemäßen Verfahrens können somit Reaktionsapparaturen aus den Werkstoffen Tantal, »Inconel 625« oder »Hastelloy C-4« eingesetzt werden, während austenitische Werkstoffe wie z. B. V4A-Stahl dafür nicht in Frage kommen.

Bei den Werkstoffen »Inconel 625« und »Hastelloy C-4« handelt es sich um bestimmte Nickelbasislegierungen (Hersteller: WIGGIN ALLOYS Ltd., Hereford, Großbritannien) folgender Zusammensetzung, angegeben in Gewichtsprozent:

| »Inconel 625« (Werkstoff Nr. 2.4856) | | »Hastelloy C-4« (Werkstoff Nr. 2.4610) | |
|---|---|---|---|
| Ni: | Rest | Ni: | Rest |
| Cr: | 20—23% | Cr: | 14—18% |
| Mo: | 8—10% | Mo: | 14—17% |
| Fe: | ≤5% | Fe: | ≤3,0% |
| Nb+Ta: | 3,15—4,15% | Co: | ≤2,0% |
| Co: | ≤1% | Ti: | 0,05—0,7% |
| Al: | ≤0,4% | C: | ≤0,015% |
| Ti: | ≤0,4% | | |
| C: | ≤0,1% | | |

**Patentansprüche**

1. Verfahren zur Herstellung von Pivalinsäurenitril, $(CH_3)_3C-CN$, dadurch gekennzeichnet, daß man Pivalinsäure mit Ammoniak, gegebenenfalls in Gegenwart von Wasserdampf, an einem Aluminiumoxid-Kontakt in der Gasphase bei Temperaturen von 370—430° C umsetzt.

2. Verfahren nach Anspruch 1, dadurch gekennzeichnet, daß Pivalinsäure und Ammoniak im Molverhältnis von 1 : 1 bis 1 : 3, bevorzugt von 1 : 1 bis 1 : 2, besonders bevorzugt von 1 : 1,05 bis 1 : 1,6 eingesetzt werden.

3. Verfahren nach Anspruch 1, dadurch gekennzeichnet, daß die Umsetzung bei einer Verweilzeit von 0,5 bis 20 Sekunden, bevorzugt von 1 bis 12 Sekunden, durchgeführt wird.

4. Verfahren nach Anspruch 1, dadurch gekennzeichnet, daß als Katalysator ein $\gamma$-Aluminiumoxid mit einer spezifischen Oberfläche von 50—350 $m^2/g$ verwendet wird.

5. Verfahren nach Anspruch 1, dadurch gekennzeichnet, daß man solche $\gamma$-Aluminiumoxid-Katalysatoren verwendet, deren Verunreinigung durch Schwermetalloxide weniger als 0,1% beträgt.

6. Verfahren nach Anspruch 1, dadurch gekennzeichnet, daß man die Umsetzung kontinuierlich durchführt.

7. Verfahren nach Anspruch 1, dadurch gekennzeichnet, daß man die Umsetzung technisch in Apparaturen durchführt, die aus Glas, Tantal oder einer Nickelbasislegierung mit ca. 56—70% Nickel, 14—23% Chrom und 8—17% Molybdän, neben geringen Mengen an anderen Elementen, gefertigt sind.

**Claims**

1. Process for the preparation of pivalonitrile, $(CH_3)_3C-CN$, characterised in that pivalic acid is reacted with ammonia, if appropriate in the presence of steam, over an aluminium oxide catalyst, in the gas phase, at temperatures of 370—430° C.

2. Process according to Claim 1, characterised in that pivalic acid and ammonia are used in a molar ratio of 1 : 1 to 1 : 3, preferably 1 : 1 to 1 : 2, particularly preferably 1 : 1.05 to 1 : 1.6.

3. Process according to Claim 1, characterised in that the reaction is carried out with a residence time of 0.5 to 20 seconds, preferably 1 to 12 seconds.

4. Process according to Claim 1, characterised in that a $\gamma$-aluminium oxide with a specific surface area of 50—350 $m^2/g$ is used as the catalyst.

5. Process according to Claim 1, characterised in that the $\gamma$-aluminium oxide catalysts used are those which are contaminated with less than 0.1% of heavy metal oxides.

6. Process according to Claim 1, characterised in that the reaction is carried out continuously.

7. Process according to Claim 1, characterised in that the reaction is carried out industrially in

apparatuses which are manufactured from glass, tantalum or a nickel-based alloy containing about 56—70% of nickel, 14—23% of chromium and 8—17% of molydenum, in addition to small amounts of other elements.

## Revendications

1. Procédé de production de nitrile d'acide pivalique $(CH_3)_3C-CN$, caractérisé en ce qu'on fait réagir de l'acide pivalique avec de l'ammoniac, éventuellement en présence de vapeur d'eau, sur un catalyseur à l'oxyde d'aluminium en phase gazeuse à des températures de 370 à 430° C.

2. Procédé suivant la revendication 1, caractérisé en ce qu'on utilise l'acide pivalique et l'ammoniac dans un rapport molaire de 1 : 1 à 1 : 3, mieux encore de 1 : 1 à 1 : 2, notamment de 1 : 1,05 à 1 : 6.

3. Procédé suivant la revendication 1, caractérisé en ce qu'on conduit la réaction pendant une durée de séjour de 0,5 à 20 secondes, de préférence de 1 à 12 secondes.

4. Procédé suivant la revendication 1, caractérisé en ce qu'on utilise comme catalyseur un oxyde d'aluminium $\gamma$ dont la surface spécifique va de 50 à 350 $m^2/g$.

5. Procédé suivant la revendication 1, caractérisé en ce qu'on utilise des catalyseurs à l'oxyde d'aluminium $\gamma$ dont la contamination par des oxydes de métaux lourds s'élève à moins de 0,1%.

6. Procédé suivant la revendication 1, caractérisé en ce qu'on conduit la réaction en continu.

7. Procédé suivant la revendication 1, caractérisé en ce qu'on conduit la réaction du point de vue technique dans des appareils qui sont fabriqués en verre, en tantale ou en alliage à base de nickel contenant environ 56—70% de nickel, 14—23% de chrome et 8—17% de molybdène, à côté de faibles quantités d'autres éléments.